# EUROPÄISCHE PATENTANMELDUNG

(11) **EP 0 860 440 A1**
(43) Veröffentlichungstag der Anmeldung: **26.08.1998**
(21) Anmeldenummer: 98102621.4
(22) Anmeldetag: 16.02.1998
(51) Int. Cl.: C07D 311/68, C07D 311/70, A61K 31/35

(54) **Sulfonamid-substituierte Chromane mit Kaliumkanal blockierender Wirkung**

(30) Priorität: 20.02.1997 DE 19706675
(71) Anmelder: HOECHST AKTIENGESELLSCHAFT, 65929 Frankfurt am Main (DE)
(72) Erfinder: Lang, Hans Jochen, Dr., 65719 Hofheim (DE); Brendel, Joachim, Dr., 61118 Bad Vilbel (DE); Gerlach, Uwe, Dr., 65795 Hattersheim (DE); Weidmann, Klaus, Dr., 61476 Kronberg (DE)

(57) **Zusammenfassung**

Chromane der Formel I 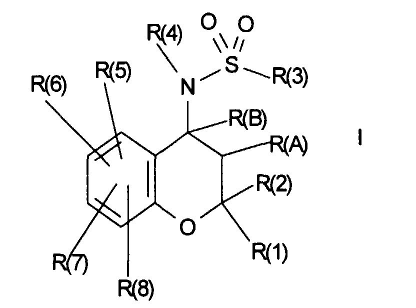 mit den im Anspruch 1 angegebenen Bedeutungen von R(A), R(B) und R(1) bis R(8) sind hervorragend geeignet zum Herstellen eines Medikaments zum Blockieren des K⁺ Kanal, der durch cyclisches Adenosinmonophosphat (cAMP) geöffnet wird; ferner zum Herstellen eines Medikaments zum Inhibieren Magensäuresekretion;
zur Behandlung von Ulcera des Magens und des intestinalen Bereiches, insbesondere des Duodenums, zur Behandlung der Refluxoesophagitis, zur Behandlung von Durchfall-Erkrankungen, zur Behandlung und Verhinderung aller Typen von Arrhythmien einschließlich atrialer, ventrikulärer und supraventrikulärer Arrhythmien, sowie zur Kontrolle von Reentry-Arrhythmien und zur Verhinderung des plötzlichen Herztodes infolge von Kammerflimmerns.

## Beschreibung

Die Erfindung betrifft Chromane der Formel I 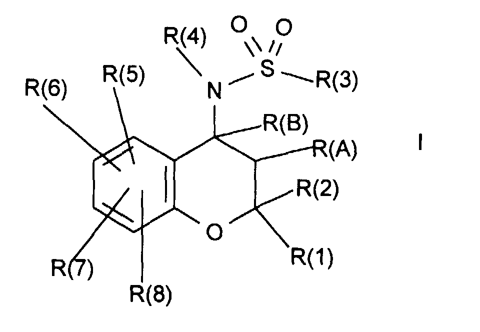 worin bedeuten:
- R(1) und R(2): unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
- R(1) und R(2): gemeinsam eine Alkylenkette mit 2, 3, 4, 5, 6, 7, 8, 9 oder 10 C-Atomen;
- R(A): Hydroxy, Alkanoyloxy mit 1, 2, 3, 4, 5 oder 6 C-Atomen oder Alkylsulfonyloxy mit 1, 2, 3, 4, 5 oder 6 C-Atomen;
- R(B): Wasserstoff;
oder
- R(A) und R(B): zusammen eine Bindung;
- R(3): R(9)-CₙH₂ₙ[NR(11)]ₘ-;
- R(9): Wasserstoff oder Cycloalkyl mit 3, 4, 5, 6, 7 oder 8 C-Atomen;
- n: Null, 1, 2, 3, 4, 5, 6, 7, 8, 9 oder 10;
- m: Null oder 1;
- R(11): Wasserstoff oder Alkyl mit 1, 2, 3, 4, 5 oder 6 C-Atomen;
oder
- R(11) und R(9): gemeinsam eine Alkylengruppe mit 1, 2, 3, 4, 5, 6, 7 oder 8 C-Atomen;
wobei eine CH₂-Gruppe der Gruppe CₙH₂ₙ durch -O-, SO_{Null, 1 oder 2}- oder -NR(10) ersetzt sein kann;
- R(10): Wasserstoff, Methyl oder Ethyl;
- R(4): R(12)-CᵣH₂ᵣ;
- R(12): Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
wobei Pyridyl, Thienyl, Imidazolyl oder Phenyl unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
- r: Null, 1, 2, 3, 4, 5, 6, 7, 8, 9, 10, 11, 12, 13, 14, 15, 16, 17, 18, 19 oder 20;
wobei eine CH₂-Gruppe der Gruppe CᵣH₂ᵣ ersetzt sein kann durch -O-, >CH=CH<, -C≡C-, -CO-, -CO-O-, SO_{Null, 1 oder 2}- oder -NR(10)-;
- R(5), R(6), R(7) und R(8): unabhängig voneinander Wasserstoff, F, Cl, Br, I, Alkyl mit 1, 2, 3, 4, 5 oder 6 C-Atomen, Cycloalkyl mit 3, 4, 5, 6, 7 oder 8 C-Atomen, -CN, -CF₃, -C₂F₅, -C₃F₇, -N₃, -NO₂, -CONR(13)R(14), -COOR(15), R(16)-CₛH₂ₛ-Y- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
- R(13) und R(14): unabhängig voneinander Wasserstoff oder Alkyl mit 1, 2 oder 3 C-Atomen;
- R(15): Wasserstoff, Methyl, Ethyl, Phenyl oder -CᵤH₂ᵤ-NR(13)R(14);
- u: 2 oder 3;
- R(16): Wasserstoff, Cycloalkyl mit 3, 4, 5, 6, 7 oder 8 C-Atomen, -COOR(15), Thienyl, Imidazolyl, Pyridyl, Chinolyl, Isochinolyl, Piperidyl, 1-Pyrrolidinyl, N-Morpholino, N-Methylpiperazino, CF₃, C₂F₅, C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
- s: Null, 1, 2, 3, 4, 5 oder 6;
- Y: -S-, -SO-, -SO₂₋, -CO-, -SO₂-NR(10)-, -O-, -NR(10)-oder -CO-NR(10);
jedoch mit der Bedingung, daß zwei der Substituenten R(5), R(6), R(7) und R(8) nicht Wasserstoff sind;
und deren physiologisch verträgliche Salze.

Bevorzugt sind Verbindungen der Formel I, in der bedeuten:
- R(1) und R(2): unabhängig voneinander Wasserstoff, Alkyl mit 1, 2 oder 3 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
- R(1) und R(2): gemeinsam eine Alkylenkette mit 4 oder 5 C-Atomen;
- R(A): Hydroxy oder Acetoxy;
- R(B): Wasserstoff;
oder
- R(A) und R(B): zusammen eine Bindung;
- R(3): R(9)-CₙH₂ₙ[NR(11)]ₘ-;
- R(9): Wasserstoff;
- n: Null, 1, 2, 3, 4, 5 oder 6;
- m: Null oder 1;
- R(11): Wasserstoff oder Alkyl mit 1, 2, 3 oder 4 C-Atomen;
- R(10): Wasserstoff, Methyl oder Ethyl;
- R(4): R(12)-CᵣH₂ᵣ;
- R(12): Wasserstoff, Cycloalkyl mit 3, 4, 5, 6, 7 oder 8 C-Atomen, CF₃, Pyridyl oder Phenyl,
wobei Pyridyl und Phenyl unsubstituiert sind oder substituiert mit 1 oder 2 Substituenten ausgewählt aus der Gruppe bestehend aus F, Cl, CF₃, Sulfamoyl und Methylsulfonyl;
- r: Null, 1, 2, 3, 4, 5, 6, 7, 8, 9 oder 10;
wobei eine CH₂-Gruppe der Gruppe CᵣH₂ᵣ ersetzt sein kann durch -O-, -CO-, -CO-O-, SO_{Null, 1 oder 2}-;
- R(5), R(6), R(7) und R(8): unabhängig voneinander Wasserstoff, F, Cl, Br, Alkyl mit 1, 2, 3 oder 4 C-Atomen, -CN, -CF₃, -NO₂, -CONR(13)R(14), -COOR(15), R(16)-CₛH₂ₛ-Y- oder Phenyl,
das unsubstituiert ist oder substituiert mit 1 oder 2 Substituenten ausgewählt aus der Gruppe bestehend aus F, Cl, und CF₃;
- R(13) und R(14): unabhängig voneinander Wasserstoff oder Alkyl mit 1, 2 oder 3 C-Atomen;
- R(15): Methyl, Ethyl, Phenyl oder -CᵤH₂ᵤ-NR(13)R(14);
- u: 2 oder 3;
- R(16): Wasserstoff, Cycloalkyl mit 5 oder 6 C-Atomen, CF₃, C₂F₅, C₃F₇ oder Phenyl,
das unsubstituiert ist oder substituiert mit 1 oder 2 Substituenten ausgewählt aus der Gruppe bestehend aus F, Cl, Br, CF₃, Methyl, Methoxy, Sulfamoyl und Methylsulfonyl;
- s: Null, 1, 2, 3 oder 4;
- Y: -S-, -SO-, -SO₂₋, -CO-, -SO₂-NR(10)-, -O-, -NR(10)- oder -CO-NR(10);
jedoch mit der Bedingung, daß zwei der Substituenten R(5), R(6), R(7) und R(8) nicht Wasserstoff sind;
und deren physiologisch verträgliche Salze.

Besonders bevorzugt sind Verbindungen der Formel I, in welcher bedeuten:
- R(1) und R(2): unabhängig voneinander Alkyl mit 1, 2 oder 3 C-Atomen;
oder
- R(1) und R(2): gemeinsam eine Alkylenkette mit 4 oder 5 C-Atomen;
- R(A): Hydroxy;
- R(B): Wasserstoff;
oder
- R(A) und R(B): zusammen eine Bindung;
- R(3): Alkyl mit 1, 2 oder 3 C-Atomen, Dimethylamino oder Diethylamino;
- R(4): R(12)-CᵣH₂ᵣ;
- R(12): Wasserstoff, Cycloalkyl mit 5 oder 6 C-Atomen oder CF₃;
- r: Null, 1, 2, 3, 4, 5, 6, 7 oder 8;
wobei eine CH₂-Gruppe der Gruppe CᵣH₂ᵣ ersetzt sein kann durch -O-, -CO-, -CO-O-, SO_{Null, 1 oder 2}-;
- R(5), R(6), R(7) und R(8): unabhängig voneinander Wasserstoff, F, Cl, Br, Alkyl mit 1 oder 2 C-Atomen, -CN, -NO₂, -COOR(15), R(16)-CₛH₂ₛ-Y- oder Phenyl,
das unsubstituiert ist oder substituiert mit 1 oder 2 Substituenten ausgewählt aus der Gruppe bestehend aus F und Cl;
- R(15): Methyl, Ethyl, Phenyl oder -CᵤH₂ᵤ-NR(13)R(14);
- u: 2 oder 3;
- R(13) und R(14): unabhängig voneinander Wasserstoff oder Alkyl mit 1, 2 oder 3 C-Atomen;
- R(16): Wasserstoff, CF₃ oder Phenyl,
- s: Null, 1, 2, 3 oder 4;
- Y: -S-,-SO-, -SO₂₋, -CO-, -SO₂-NR(10)-, -O-, -NR(10)- oder -CO-NR(10);
- R(10): Wasserstoff oder Methyl;
jedoch mit der Bedingung, daß zwei der Substituenten R(5), R(6), R(7) und R(8) nicht Wasserstoff sind;
und deren physiologisch verträgliche Salze.

Enthalten die Verbindungen I eine saure oder basische Gruppe bzw. einen basischen Heterocyclus, so sind auch die entsprechenden, pharmakologisch und toxikologisch verträglichen Salze Gegenstand der Erfindung. So können die Verbindungen I, die eine oder mehrere -COOH-Gruppen tragen, beispielsweise als Alkalisalze, vorzugsweise als Natrium- oder Kaliumsalze verwendet werden. Verbindungen I, die eine basische, protonierbare Gruppe oder einen basischen heterocyclischen Rest tragen, können auch in Form ihrer organischen oder anorganischen, pharmakologisch und toxikologisch verträglichen Säureadditionssalze verwendet werden, beispielweise als Hydrochloride, Methansulfonate, Acetate, Lactate, Maleinate, Fumarate, Malate, Gluconate usw. Enthalten die Verbindungen I eine saure und basische Gruppe im gleichen Molekül, so gehören neben den geschilderten Salzformen auch innere Salze, sogenannte Betaine, zur der Erfindung.

Enthalten die Substituenten der Verbindungen I Gruppen mit unterschiedlichen stereochemischen Möglichkeiten, so gehören auch die einzelnen möglichen Stereoisomeren zur Erfindung. So enthalten die Verbindungen I chirale Zentren in Position 3 und 4 des Chromansystems, so daß die einzelnen reinen optischen Isomeren sowie beliebige Stoffgemische der optischen Isomeren Teil der Erfindung sind.

Die Verbindungen der Formel I sind durch unterschiedliche chemische Verfahren herstellbar, die ebenfalls Teil der Erfindung sind.

So erhält man eine Verbindung der Formel I, indem man
a) eine Verbindung der Formel II 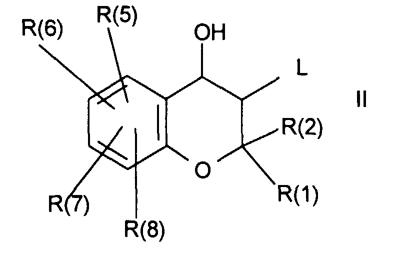 worin R(1), R(2), R(5), R(6), R(7) und R(8) die angegebene Bedeutung besitzen und L die für eine Alkylierung übliche nucleofuge Fluchtgruppe, insbesondere Cl, Br oder I, bedeutet, mit einem Sulfonamid oder dessen Salz der Formel III 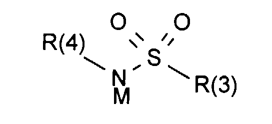 in an sich bekannter Weise umsetzt, worin R(3) und R(4) die angegebene Bedeutung besitzen und M für Wasserstoff oder für ein Metallatom, besonders bevorzugt für Lithium, Natrium oder Kalium steht;
   oder daß man
b) ein Epoxid der Formel IV 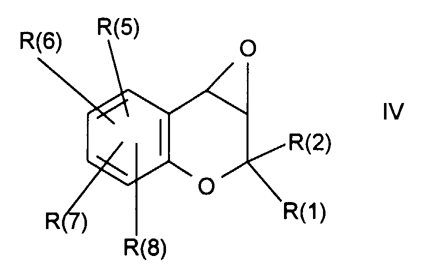 worin R(1), R(2), R(5), R(6), R(7) und R(8) die angegebene Bedeutung besitzen, mit einem Sulfonamid oder dessen Salz der Formel III 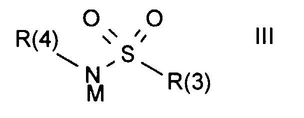 in an sich bekannter Weise umsetzt, worin R(3) und R(4) die angegebene Bedeutung besitzen und M für Wasserstoff oder für ein Metallatom, besonders bevorzugt für Lithium, Natrium oder Kalium steht;
   oder daß man
c) eine Verbindung der Formel V 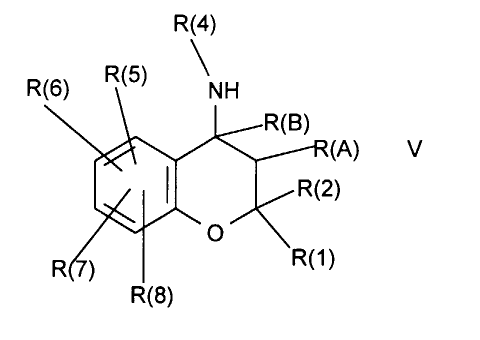 worin R(1), R(2), R(4), R(5), R(6), R(7), R(8), R(A) die angegebene Bedeutung besitzen und R(B) und R(A) gleich OH und R(B) gleich Wasserstoff ist, mit einem Sulfonsäure-Derivat der Formel VI

   R(3) - SO₂-W VI

   umsetzt, worin R(3) die angegebene Bedeutung besitzt und W eine nucleofuge Fluchtgruppe, wie Fluor, Brom, 1-Imidazolyl, insbesondere aber Chlor bedeutet;
   oder daß man
d) eine Verbindung der Formel VII 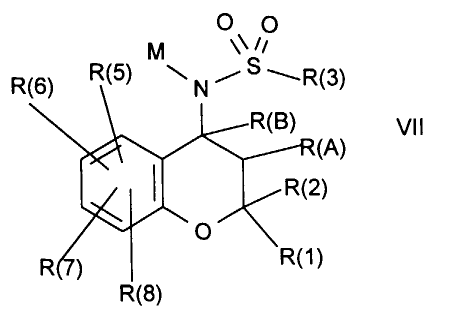 worin R(1), R(2), R(3), R(5), R(6), R(7), R(8), R(A), R(B) und M die angegebene Bedeutung besitzen, in an sich bekannter Weise mit einem Alkylierungsmittel der Formel VIII

   R(4)-L VIII

   im Sinne einer Alkylierungsreaktion umsetzt, worin R(4) mit Ausnahme von Wasserstoff sowie L die angegebene Bedeutung besitzen;
   oder daß man
e) in einer Verbindung der Formel I 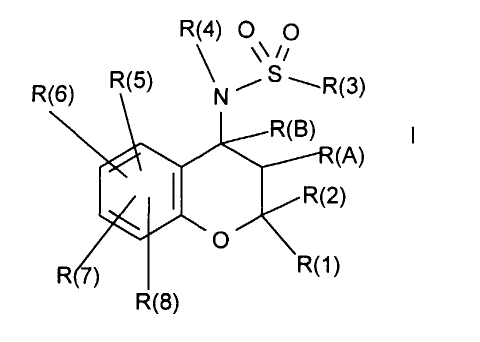 worin R(1) bis R(4) die angegebene Bedeutung besitzen, in mindestens einer Position R(5) bis R(8) eine aromatische Substitutionsreaktion durchführt;
f) oder daß man Verbindungen der Formel I, worin R(1) bis R(8) und R(A) die angegebene Bedeutung besitzen und R(B) Wasserstoff bedeutet, durch eliminierende Abspaltung von R(A) und R(B) in Chromene der allgemeinen Formel I überführt, worin R(A) und R(B) eine Bindung bedeuten und die restlichen Substituenten die angegebene Bedeutung besitzen.

### Verfahrensweise a)

beschreibt die an sich bekannte Alkylierung eines Sulfonamids bzw. eines seiner Salze der Formel III durch ein alkylierend wirkendes Chromanderivat der Formel II. Da die Alkylierung eines Sulfonamides aus der Salzform heraus erfolgt, muß bei Verwendung eines freien Sulfonamids (Formel III, M = H) durch Einwirkung einer Base ein Sulfonamidsalz (Formel III, M = Kation) erzeugt werden, das sich durch höhere Nucleophilie und damit durch höhere Reaktivität auszeichnet. Setzt man freies Sulfonamid (M = H) ein, so erfolgt die Deprotonierung des Sulfonamids zum Salz in situ unter bevorzugter Verwendung solcher Basen, die selbst nicht oder nur wenig alkyliert werden, wie Natriumcarbonat, Kaliumcarbonat, ein sterisch stark gehindertes Amin, z. B. Dicyclohexylamin, N,N,N-Dicyclohexyl-ethylamin oder andere starke Stickstoffbasen mit geringer Nucleophilie, beispielsweise DBU, N,N',N''-Triisopropylguanidin etc. Es können allerdings auch andere üblich verwendete Basen für die Reaktion eingesetzt werden, wie Kalium-tert.butylat, Natriummethylat, Alkalihydrogencarbonate, Alkalihydroxide, wie beispielweise LiOH, NaOH oder KOH, oder Erdalkalihydroxide, beispielsweise Ca(OH)₂.

Dabei arbeitet man vorzugsweise in aprotischen polaren Lösungsmitteln wie Dimethylformamid, Dimethylacetamid, Tetramethylharnstoff, Hexamethylphosphorsäuretriamid, Tetrahydrofuran usw. Prinzipiell kann aber auch in polaren protischen Lösungsmitteln gearbeitet werden, wie Wasser, Methanol, Ethanol, Isopropanol, Ethylenglycol oder dessen Oligomeren und deren entsprechenden Halbethern und Ethern. Die Reaktion wird in einem bevorzugten Temperaturbereich von 20 bis 140°C, besonders bevorzugt von 40 bis 100°C durchgeführt. In günstiger Weise kann Verfahrensweise a) auch unter den Bedingungen einer Zweiphasenkatalyse durchgeführt werden.

Die Verbindungen II mit L gleich Br erhält man beispielweise in literaturbekannter Weise aus einem Chromen der Formel (XI) 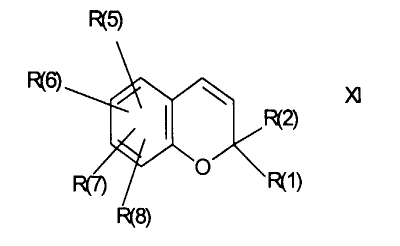 mit NBC in Gegenwart von NaOH.

Die genannten Chromene der Formel (XI) erhält man in Analogie zur Literatur beispielsweise durch Eliminierung von Wasser aus 4-Chromanolen der Formel (XII) 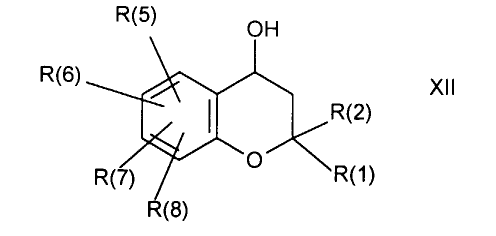 durch Einwirkung einer Säure, beispielsweise von Schwefelsäure, oder durch literaturbekannte thermische Cyclisierung von Alkinethern der Formel (XIII)

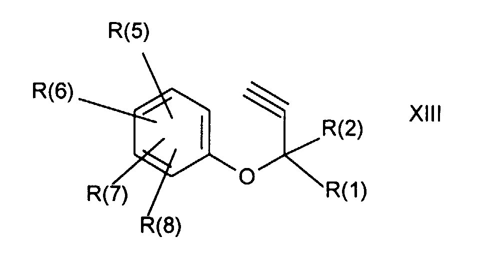

Die 4-Chromanole erhält man in literaturbekannter Weise durch Reduktion der entsprechenden Chromanone (XIV), 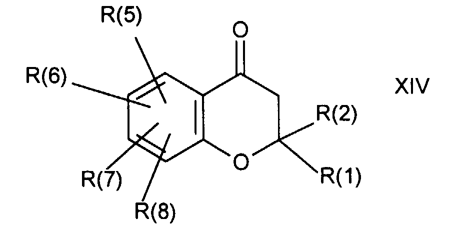 die man in bekannter Reaktion aus den Acetophenon-Derivaten der Formel (XV) durch cyclisierende Aldolkondensation 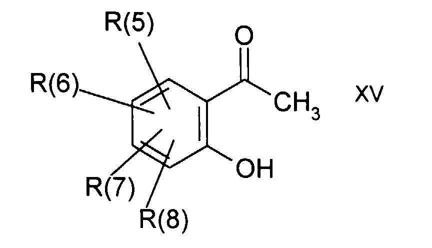 mit einem Keton (XVI) 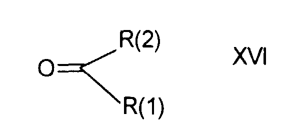 erhält.

Die Verbindungen der Formel III erhält man bevorzugt durch Umsetzung des entsprechenden Sulfonsäurechlorides mit einem Amin. Die Herstellung von Sulfonamiden ist hinreichend in der wissenschaftlichen Literatur wie auch in der Patentliteratur beschrieben

### Verfahrensweise b)

beschreibt die an sich bekannte und häufig angewandte Umsetzung eines Epoxids der Formel IV mit einem Sulfonamid der Formel III bzw. einem seiner Salze. Besonders günstig hat sich dabei die Verwendung des freien Sulfonamids III in Gegenwart katalytischer Mengen eines seiner Salze, beispielsweise des entsprechenden Na⁺-Salzes erwiesen, das man in einfacher literaturbekannter Weise wiederum durch Zugabe einer kleinen Menge einer ausreichend starken Base, z. B. Natriumhydrid zu einer Lösung oder Suspension des Sulfonamids vorteilhaft in situ erzeugen kann. Man arbeitet bei dieser Umsetzung vorteilhaft in den unter Verfahrensweise a) beschriebenen Lösungsmitteln und in dort beschriebenen Temperaturbereichen. Die Epoxide der allgemeinen Formel IV gewinnt man in literaturbekannter Weise aus der entsprechenden 3,4-Dehydrochromanen durch Epoxidierung oder durch Abspaltung von HL aus Verbindungen der Formel II mit Basen.

### Verfahrensweise c)

beschreibt die an sich bekannte und häufig angewandte Reaktion einer aktivierten Sulfonylverbindung der Formel VI, insbesondere einer Chlorsulfonylverbindung (W = Cl), mit einem Amin der allgemeinen Formel V zum entsprechenden Sulfonamid-Derivat der allgemeinen Formel I. Die Reaktion kann prinzipiell ohne Lösungsmittel durchgeführt werden, jedoch werden derartige Reaktionen in den meisten Fällen unter Verwendung eines Lösungsmittels durchgeführt.

Die Reaktionsführung geschieht vorzugsweise unter Verwendung eines polaren Lösungsmittels vorzugsweise in Gegenwart einer Base, die selbst als Lösungsmittel verwendet werden kann, z.B. bei Verwendung von Triethylamin, Pyridin und dessen Homologen. Vorzugsweise verwendete Lösungsmittel sind beispielsweise Wasser, aliphatische Alkohole, z.B. Methanol, Ethanol, Isopropanol, sek. Butanol, Ethylenglykol und dessen monomere und oligomere Monoalkyl- und Dialkylether, Tetrahydrofuran, Dioxan, dialkylierte Amide wie DMF, DMA, sowie TMU und HMPT. Man arbeitet dabei bei einer Temperatur von 0 bis 160°C, vorzugsweise von 20 bis 100°C.

Die Amine der Formel V erhält man in an sich literaturbekannter Weise bevorzugt aus den entsprechenden Verbindungen der Formel II oder den Epoxiden der Formel IV, indem man entweder mit Ammoniak oder einem Amin der Formel IX

R(4)-NH₂ IX

mit R(4) in der angegebenen Bedeutung zur Reaktion bringt.

### Verfahrensweise d)

beschreibt wie auch Verfahrensweise a) die an sich bekannte Alkylierungsreaktion eines Sulfonamids bzw. eines seiner Salze VII mit einem Alkylierungsmittel der Formel VIII. Entsprechend dieser Reaktionsanalogie gelten für Verfahrensweise d) die bereits ausführlich unter Verfahrensweise a) beschriebenen Reaktionsbedingungen.

Die Herstellung der Sulfonamid-Derivate VII und deren Vorprodukte wurde bereits bei Verfahrensweise c) beschrieben. Die Herstellung der Alkylantien VII erfolgt nach Analogvorschriften der Literatur bzw. wie unter Verfahrensweise a) beschrieben, vorzugsweise aus den entsprechenden Hydroxyverbindungen (Formel VII mit L gleich -OH).

### Verfahrensweise e)

beschreibt die weitere chemische Umwandlung von erfindungsgemäßen Verbindungen der Formel I in andere Verbindungen der Formel I beispielsweise durch elektrophile Substitutionsreaktionen in einer oder in mehreren der mit R(5) bis R(8) bezeichneten Positionen. Für den Fall, daß einer oder zwei Substituenten der Reste R(5) bis R(8) Wasserstoff bedeuten, kann in bevorzugter und literaturbekannter Weise eine elektrophile aromatische Substitutionsreaktion durchgeführt werden.

Bevorzugte elektrophile Substitutionsreaktionen sind
1. die aromatische Nitrierung zur Einführung einer oder mehrerer Nitrogruppen, sowie deren nachfolgende Reduktion zu NH₂-,
2. die aromatische Halogenierung insbesondere zur Einführung von Chlor, Brom oder Jod,
3. die Chlorsulfonierung zur Einführung einer Chlorsulfonylgruppe durch Einwirkung von Chlorsulfonsäure,
4. die Friedel-Crafts Acylierungsreaktion zur Einführung eines Acylrestes R(16)-CₛH₂ₛ-CO- oder eines Sulfonylrestes R(16)-CₛH₂ₛ-SO₂- durch Einwirkung der entsprechenden Säurechloride R(16)-CₛH₂ₛ-CO-Cl bzw. R(16)-CₛH₂ₛ-SO₂-Cl in Gegenwart einer Lewis-Säure als Friedel-Crafts-Katalysator, vorzugsweise von wasserfreiem Aluminiumchlorid.

Außerdem können mit Hilfe palladiumorganischer Synthesen in literaturbekannter Weise zahlreiche Gruppen beispielsweise unter Austausch von aromatisch gebundenem Brom in einem der Substituenten R(5), R(6), R(7) oder R(8) eingeführt werden, wie z.B. Alkinyl-, Alkenyl, Alkyl, Anilino, Phenoxyreste usw.

### Verfahrensweise f)

beschreibt die Überführung eines Chromanols der Formel I mit R(A) gleich OH in ein Chromen der Formel I, worin R(A) und R(B) eine gemeinsame Bindung bilden. Hierzu kann aus dem Chromanol entweder direkt in Gegenwart einer Säure oder einer Base Wasser abgespalten werden, oder es kann zunächst eine Aktivierung der Hydroxygruppe R(A) erfolgen, beispielsweise durch Acylierung mit einem Alkansäureanhydrid mit 3 - 12 C-Atomen.

Auf diese Weise lassen sich auch die erfindungsgemäßen Verbindungen der Formel I herstellen, worin R(1) bis R(5) die angegebene Bedeutung besitzen, R(B) Wasserstoff und R(A) einen Alkanoylrest mit 1 bis 6 C-Atomen bedeutet. Die anschließende Eliminierung zum Chromentyp der Formel I, worin R(A) und R(B) eine gemeinsame Bindung bedeuten, erreicht man vorzugsweise unter basenkatalysierten Bedingungen, z.B. durch Erhitzen mit DBU (Diazabicycloundecen).

Die Verbindungen I sind strukturverwandt mit der in der letzten Dekade in der Arzneimittelchemie intensiv bearbeiteten Klasse der 4-Acylaminochroman-Derivate, insbesondere von 2,2-Dialkyl-4-acylamino-3-chromanolen.

Prominentester Vertreter derartiger 4-Acylaminochromane ist das Cromakalim der Formel X 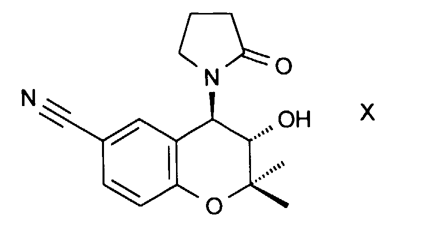 und zahlreiche, sich von diesem Präparat ableitenden Folgepräparate (z.B. Edwards and Weston, TIPS 11, 417-422 (1990), "Structure Activity Relationships of K⁺ channel openers".

Bei Cromakalim und anderen verwandten 4-Acylaminochroman-Derivaten handelt es sich um Verbindungen mit relaxierender Wirkung auf glattmuskuläre Organe, so daß sie zur Senkung des erhöhten Blutdruckes infolge Gefäßmuskelrelaxation und in der Behandlung des Asthmas infolge der Relaxation der glatten Muskulatur der Atemwege verwendet werden. All diesen Präparaten ist gemeinsam, daß sie auf zellulärer Ebene beispielsweise von glatten Muskelzellen wirken und dort zu einer Öffnung bestimmter ATP-sensitiver K⁺-Kanäle führen. Der durch den Austritt von K⁺-Ionen induzierte Anstieg von negativer Ladung in der Zelle ("Hyperpolarisation") wirkt über Sekundärmechanismen der Erhöhung von intrazellulärem Ca²⁺ und damit einer Zellaktivierung, z. B. einer Muskelkontraktion, entgegen.

Im Gegensatz zu diesen 4-Acylaminochroman-Derivaten, die wie erwähnt als Öffner des ATP-sensitiven K⁺ Kanals identifiziert wurden, zeigen die erfindungsgemäßen Verbindungen der Formel I mit der 4-Sulfonylaminostruktur überraschenderweise eine starke und spezifische blockierende (schließende) Wirkung auf einen K⁺ Kanal, der durch cyclisches Adenosinmonophosphat (cAMP) aktiviert wird und sich grundlegend vom erwähnten K⁺(ATP)-Kanal unterscheidet. Neuere Untersuchungen zeigen, daß dieser am Dickdarm identifizierte K⁺(cAMP) -Kanal sehr ähnlich oder möglicherweise identisch mit dem am Herzmuskel identifizierten I_{Ks} - Kanal ist. Infolge dieser Blockierung des K⁺(cAMP) -Kanals (= I_{Ks}-Kanals) entwickeln die Verbindungen im lebenden Organismus pharmakologische Wirkungen von hoher therapeutischer Verwertbarkeit.

So zeichnen sich die Verbindungen als neue Wirkstoffklasse potenter Inhibitoren der stimulierten Magensäuresekretion aus. Die Verbindungen der Formel I sind somit wertvolle Medikamente zur Behandlung von Ulcera des Magens und des intestinalen Bereiches, beispielsweise des Duodenums, aus. Sie eignen sich ebenfalls infolge ihrer starken magensaftsekretionshemmenden Wirkung als ausgezeichnete Therapeutika zur Behandlung der Refluxoesophagitis.

Die Verbindungen zeichnen sich weiterhin durch eine antidiarrhoische Wirkung aus und sind deshalb als Arzneimittel zur Behandlung von Durchfall-Erkrankungen geeignet.

Weiterhin können die Verbindungen I als Arzneimittel zur Behandlung und Verhinderung aller Typen von Arrhythmien einschließlich atrialer, ventrikulärer und supraventrikulärer Arrhythmien verwendet werden. Sie können insbesondere angewandt werden zur Kontrolle von Reentry-Arrhythmien und zur Verhinderung des plötzlichen Herztodes infolge von Kammerflimmern.

Inzwischen existieren Publikationen, in denen eine Korrelation zwischen I_{Ks}-Kanal-inhibitorischer Wirkung und dem Unterbinden von lebensbedrohlichen cardialen Arrhythmien beschrieben wird, wie sie beispielsweise durch β-adrenerge Hyperstimulation ausgelöst werden [z. B. T. J. Colatsky, C. H. Follmer und C .F. Starmer: *"*Channel Specificity in Antiarrhythmic Drug Action; Mechanism of potassium channel block and its role in suppressing and aggravating cardiac arrhythmias*"*, Circulation (1990) 82: 2235 - 2242; A. E. Busch, K. Malloy, W. J. Groh, M. D. Varnum, J. P. Adelman und J. Maylie; *"*The novel class III antiarrhythmics NE-10064 and NE-10133 inhibit I_{sK} channels in xenopus oocytes and I_{Ks} in guinea pig cardiac myocytes*"*, Biochem. Biophys. Res. Commun. (1994) 202: 265 - 270].

Neben den oben genannten Cromakalim- bzw. Acylaminochroman-Derivaten wurden im Verlauf der letzten Jahre in der Literatur auch Verbindungen mit 4-Sulfonylaminochroman-Struktur beschrieben, die sich entweder in der Struktur oder in der biologischen Wirkung von den erfindungsgemäßen Verbindungen der Formel I deutlich unterscheiden. So beschreibt die Europäische Offenlegungsschrift 315 009 Chromanderivate mit 4-Phenylsulfonylamino-Struktur, die sich durch antithrombotische und antiallergische Eigenschaften auszeichnen.

Die Europäische Offenlegungsschrift 370 901 beschreibt 3-Hydroxychromanderivate mit einer 4-Phenylsulfonylaminogruppe, worin die restliche Valenz des N-Atoms ein Wasserstoffatom trägt. Diese Verbindungen sind somit in essentiellen Gruppen von der Formel I bzw. Ia unterschiedlich substituiert. Entsprechend werden für diese Verbindungen der Europäischen Offenlegungsschrift 370 901 Wirkungen auf das Zentralnervensystem gefunden, so daß sie sich auch in pharmakologischer Hinsicht unterscheiden.

Die Europäische Offenlegungsschrift 389 861 beschreibt 3-Hydroxychromanderivate mit 4-Sulfonylaminogruppe. Dabei handelt es sich bei den in der Patentschrift beschriebenen Benzopyranderivaten um Aktivatoren bzw. Öffner des sogenannten adenosintriphosphat-sensitiven K⁺-Kanals [K⁺(ATP) -Kanal]. Die pharmakologischen Wirkungen von K⁺(ATP) -Kanalöffnern sind nun bekanntermaßen vollständig verschieden von den hier beschriebenen Blockern des I_{Ks}-Kanals. So wurden für die K⁺(ATP) -Kanalöffner vasodilatierende und blutdrucksenkende Eigenschaften nachgewiesen, wie sie für diesen Mechanismus typisch sind. Die von den Autoren synthetisierten, beschriebenen K⁺(ATP) -Kanalöffner zeigen erwartungsgemäß für diesen Mechanismus der K⁺-Kanalöffnung typische, spezielle antiarrhythmische Eigenschaften, die sich von den antiarrhythmischen Eigenschaften der Verbindungen der Formel I ganz grundsätzlich insbesondere hinsichtlich des Typs der zu behandelnden Arrhythmien unterscheiden. In einer grundlegenden Arbeit konnte von Lucchesi et al. ( J.Cardiovasc. Pharmacol. 15, 452 - 464 [1990]) eindrucksvoll gezeigt werden, daß K⁺(ATP) -Kanalöffner am sauerstoffunterversorgten kranken Herzen oder bei plötzlichen schämen nicht antiarrhythmisch wirken, sondern sogar im Gegenteil lebensbedrohliche profibrillatorische Effekte verursachen. Diese gefährlichen Zustände werden als Konsequenz der aus der Aktivierung des K⁺(ATP) -Kanals resultierenden Verkürzungen der Repolarisationsdauer ausgelöst. Im Gegensatz zu diesen am kranken, mangelversorgten Herzen lebensbedrohlichen profibrillatorischen Effekten durch die Wirkung von K⁺(ATP) -Kanalöffnern sollten Blocker des K⁺(cAMP)-Kanals unter diesen Bedingungen antifibrillatorische Wirkung zeigen. Als prominenter Vertreter eines strukturverwandten Chromanols fand inzwischen 6-Cyano-4-(N-ethylsulfonyl-N-methylamino)-2,2-dimethyl-3-chromanol Eingang in die neueste Literatur als Beispiel eines hochspezifischen Iₖₛ₋ bzw. I_{sK} -Kanalblockers mit einer entsprechenden Verlängerung des Aktionspotentials am Herzen (Süßbrich et al, Naunyn Schiedebergs Arch. Pharm. (1996) 353 (4. Suppl.), R72; Pflügers Arch.-Eur. J. Physiol. 431( 6) [Suppl], R 22 (1996), A. Busch et al., Pflügers Arch.-Eur. J. Physiol. 432 (6) [Suppl], 1094-1096 (1996)). Gegenstand der Erfindung ist deshalb auch die Verwendung von Verbindungen der Formel I zur Behandlung des Sudden Cardiac Death, ventrikulärer Fibrillationen und generell von Arrhythmien des kranken Herzen, die auf den I_{Ks}-Kanals zurückzuführen sind.

Die in vorliegender Erfindung beschriebenen Verbindungen unterscheiden sich von potentiell denkbaren, unter die Ansprüche der EP-OS 389 861 fallenden Verbindungen sowie von den in der Literatur (Pflügers Arch. - Eur. J. Physiol. (1995) 429: 517 - 530. A new class of inhibitors of cAMP-mediated Cl-secretion in rabbit colon, acting by the reduction of cAMP-activated K⁺ conductance) beschriebenen Chromanolen dadurch, daß mindestens zwei der Substituenten der Reste R(5), R(6), R(7) und R(8) nicht Wasserstoff bedeuten.

Diese speziellen, von uns hergestellten und untersuchten Verbindungen der Formel I zeichnen sich durch ein überlegenes pharmakologisches Wirkprofil aus, wie beispielsweise durch eine höhere potency und/oder durch eine höhere Organselektivität und/oder durch günstigere pharmakokinetische Eigenschaften, z. B. durch eine verlängerte Halbwertszeit aus.

Die Publikation "N-sulfonamides of benzopyran-related potassium channel openers: conversion of glyburyde insensitive smooth muscle relaxants to potent smooth muscle contractors*"* in Bioorg. Med. Chem. Lett. (1994) 4: 769 - 773 beschreibt spezielle trifluormethylsubstituierte 4-Sulfonylaminochroman-Derivate, die aber im Gegensatz zu den hier beschriebenden strukturell andersartigen K⁺(cAMP)-Kanalblockern biologisch andersartige pharmakologische Wirkungen und damit andere therapeutische Anwendungsbereiche besitzen.

In jüngster Zeit wurden außerdem Spiro[2H-1-benzopyran-2,4'-piperidine] mit essentieller basischer Seitengruppe in der Literatur beschrieben, z. B. MK-499 *"*Cardiac electrophysiology and antiarrhythmic actions of two long-acting spirobenzopyran piperidine class III agents, L-702,958 and L-706,000 (MK 499)*"* J. Pharmakol. Exp. Ther. (1994) 269: 541 - 554; T. J. Colatsky und T. M. Argentieri, *"*Potassium channel blockers as antiarrhythmic drugs*"*; Drug Develp. Res. (1994) 33: 235 - 249. Diese *"*Spirobenzopyran-Piperidines Class III Agents*"* werden allerdings in der Literatur hinsichtlich ihrer Wirkungsweise sehr klar charakterisiert [P. S. Spector, M. E. Curran, M. T. Keating, M. C. Sanguinetti, Circulation Res. (1996) 78: 499 - 503; J. J. Lynch et al., J. Pharmacol. Exp. Ther. (1994) 269: 541 - 554]. Dabei wird in der zitierten Literatur eindeutig beschrieben und gezeigt, daß die antiarrhythmische Wirkung dieser Verbindungen durch die Inhibition des HERG-Kanals und der rasch (rapid) aktivierendenden Komponente des delayed rectifyer K⁺ -Kanals, des I_{Kr}-Kanals, verursacht wird. Damit sind die Spirobenzopyran-Piperidine als Substanzen mit proarrhythmischer Komponente und mit der Gefahr einer erhöhten Mortalität gegen Placebo gekennzeichnet, wie sie für diese Wirkstoffklasse in der Sword Studie gezeigt wurde. Dies steht im klaren Gegensatz zu den hier beschriebenen Verbindungen, deren Vorteil in der Blockierung der langsam (slow) aktivierendenden Komponente des delayed rectifyer K⁺-Kanals, des I_{Ks}-Kanals, besteht, die diese proarrhythmische Komponente nicht besitzen.

Arzneimittel, die eine erfindungsgemäße Verbindung I enthalten, können oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes des Formel I in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen; aber auch von Art und Stärke der zu behandelnden Krankheit sowie von Geschlecht, Alter, Gewicht und individueller Ansprechbarkeit des zu behandelnden Säugers ab.

Im Durchschnitt beträgt die tägliche Dosis einer Verbindung der Formel I bei einem etwa 75 kg schweren Patienten mindestens 0.001 mg, vorzugsweise mindestens 0.01 mg, insbesondere mindestens 0,1 mg, bis höchstens 1 g, vorzugsweise bis 100 mg, insbesondere bis höchstens 10 mg pro kg Körpergewicht.

Erklärung der im Text verwendeten Abkürzungen
- DMSO: Dimethylsulfoxid
- NBS: N-Bromsuccinimid
- M: mol

### Beispiel 1: 6-Cyano-4-[N-ethylsulfonyl-N-methylamino]-7-fluor-2,2-dimethyl-3-chromanol

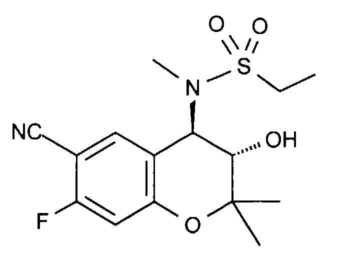

a) 10 g (0,072 M) 2-Fluor-4-hydroxybenzonitril, 0,072 M 1-Chlor-1,1-dimethylpropin und 15 g (entsprechend 16.3 ml oder 0.036 M) Benzyltrimethylammonium-hydroxid (in Methanol, 40%ig) werden in 80 ml Methylenchlorid suspendiert und mit einer Lösung aus 4.32 g (0.108 M) NaOH in 80 ml Wasser versetzt und 3 Tage bei Raumtemperatur gerührt. Die Phasen werden getrennt und die wäßrige Phase mehrfach mit Methylenchlorid extrahiert. Man wäscht die organische Lösung mit 10%iger NaOH und anschließend mit Wasser und destilliert das organische Lösungsmittel unter verminderten Druck ab. Man erhält 4-(1,1-dimethyl-1-propinyloxy)-2-fluor-benzonitrilals rotgefärbtes öliges Produkt.
b) 9,6 g (0,047 M) 4-(1,1-dimethyl-1-propinyloxy)-2-fluorbenzonitril werden in etwa 30 ml 1,2-Dichlorbenzol unter Argon für 6 Stunden unter magnetischer Rührung auf 170 °C erhitzt. Man destilliert das Lösungsmittel ab und chromatografiert den amorphen Rückstand an Kieselgel mit einem Gemisch aus 1 Teil Ethylacetat und 3 Teilen Toluol als Elutionsmittel. Man erhält 6-Cyano-7-fluor-2,2-dimethyl-3,4-chromen als zähes Öl.
c) 2,03 g (0,01 M) 6-Cyano-7-fluor-2,2-dimethyl-3,4-chromen werden in einer Mischung aus 8 ml DMSO und 0,3 ml Wasser gelöst und unter intensiver Außenkühlung portionsweise mit 3,54 g (0,02 M) N-Bromsuccinimid so zugefügt, daß die Temperatur möglichst unter 20°C, auf jeden Fall aber unter 30°C gehalten werden soll. Nach dem Rühren für weitere 3 Stunden bei 15 - 20°C Stunden gießt man auf eine Suspension aus Eis und Wasser, extrahiert die wäßrige Phase mit Ethylacetat und trocknet über wasserfreiem Natriumsulfat. Nach Abdestillieren des Lösungsmittels chromatographiert man den amorphen Rückstand an Kieselgel mit einem Gemisch aus Toluol / Essigester (3 : 1) als Elutionsmittel. Kristallisation in Gegenwart von n-Heptan und wenig Diisopropylether. Man erhält trans-4-Brom-6-cyano-7-fluor-3-hydroxy-2,2-dimethylchroman als bräunlich gefärbten kristallinen Feststoff, Schmp. 75 - 82°C.
d) Zu einer Lösung aus 1,2 g (0.0039 M) trans-4-Brom-6-cyano-7-fluor-3-hydroxy-2,2-dimethylchroman in 40 ml wasserfreiem Tetrahydrofuran gibt man 1,05 g (0.026 M) gemahlenes Natriumhydroxid und rührt die Suspension 3 Stunden bei Raumtemperatur. Man filtriert vom Niederschlag ab und wäscht die organische Phase mit einer wäßrigen Kochsalzslösung. Nach dem Verdampfen des organischen Lösungsmittels im Vakuum erhält man 6-Cyano-7-fluor-2,2-dimethyl-3,4-epoxychroman als amorphen Rückstand, der in Gegenwart von n-Heptan kristallisiert. Schmp. 68 - 70 °C.
e) 0.46 g (0,0037 M) Ethylsulfonsäure-N-methylamid werden in 10 ml wasserfreiem DMSO gelöst und unter Argon zu einer Suspension von 0,026 g (0.00088 M) Natriumhydrid (60%ige Suspension in Öl) zugetropft, wobei die Reaktion unter Bildung von Wasserstoff abläuft. Zu der so erhaltenen Mischung tropft man eine Lösung aus 0.65 g (0.0029 M) 6-Cyano-7-fluor-2,2-dimethyl-3,4-epoxychroman in 10 ml DMSO und rührt das Reaktionsgemisch über 6 Stunden bei 50°C und weitere 14 Stunden bei Raumtemperatur. Nach Zugabe von Wasser extrahiert man mehrmals mit Ethylacetat, wäscht die organischen Phasen mit Wasser, trocknet über wasserfreiem Natriumsulfat und destilliert das Lösungsmittel ab. Man chromatografiert den amorphen Rückstand an Kieselgel mit einem Gemisch aus 1 Teil Ethylacetat und 3 Teilen Toluol als Elutionsmittel und destilliert das Elutionsmittel ab. Nach Kristallisation unter Diisopropylether erhält man 6-Cyano-4-[N-ethylsulfonyl-N-methylamino]-7-fluor-2,2-dimethyl-3-chromanol als farblosen Feststoff. Schmp. 175 - 180 °C.

### Beispiel 2: 7-Chlor-6-cyano-4-[N-ethylsulfonyl-N-methylamino]-2,2-dimethyl-3-chromanol

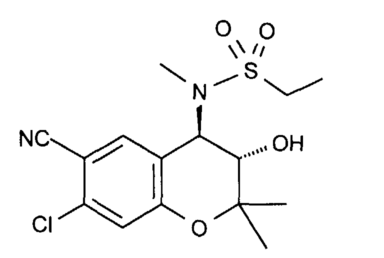

a) 5,1 g (0,026 M) 4-Acetoxy-2-chlorbenzonitril (dargestellt durch Erhitzen von 2-Chlor-4-hydroxybenzonitril in Acetanhydrid, Schmp. 76 - 78°C) werden mit 10,43 g (0,078 M) wasserfreiem Aluminiumchlorid als Friedel-Crafts-Katalysator versetzt und die Mischung der beiden Feststoffe unter mechanischer Rührung auf 120 - 130°C über 60 - 90 Minuten erhitzt. Der amorphe Rückstand wird vorsichtig mit Wasser zersetzt und die wäßrige Suspension 1 Stunde gerührt. Man filtriert das 4-Chlor-5-cyano-2-hydroxy-acetophenon ab und kristallisiert aus einer Mischung von Essigester und Diisopropylether. Farblose Kristalle, Schmp. 183 - 187 °C.
b) Eine Mischung aus 2,5 g (0,0127 M) 4-Chlor-5-cyano-2-hydroxy-acetophenon in 100 ml Toluol versetzt man mit 1,8g (0,0317 M) Aceton und 0,23g (0,033 M) Pyrrolidin und kocht über 3 Stunden am aufgesetzten Wasserabscheider unter Rückfluß. Nach Zugabe von weiteren 2,3 ml Aceton und 0,1 ml Pyrrolidin wird 6 Stunden am Wasserabscheider gekocht. Das Lösungsmittel wird abdestilliert, der Rückstand mit Wasser versetzt und mit Ethylacetat extrahiert. Nach Waschen der organischen Phase mit 2 N HCl und Wasser wird das Lösungsmittel abdestilliert und das amorphe 7-Chlor-6-cyano-2,2-dimethyl-4-chromanon unter Diisopropylether zur Kristallisation gebracht. Schmp. 88 - 95 °C.
c) In eine Mischung aus 5.89 g (0,025 M) 7-Chlor-6-cyano-2,2-dimethylchromanon in 120 ml Ethanol trägt man portionsweise unter magnetischer Rührung 1,32 g (0,035 M) Natriumboranat ein. Man rührt die Lösung 4 Stunden bei Raumtemperatur, destilliert das Lösungsmittel ab, versetzt den Rückstand mit Wasser und stellt mit 2 N HCl auf pH 6 - 7. Die Suspension wird etwa 2 Stunden bei Raumtemperatur gerührt und das kristalline 7-Chlor-6-cyano-2,2-dimethyl-4-chromanol abfiltriert. Schmp. 88 - 92 °C.
d) Eine Mischung aus 5,1 g (0,021 M) 7-Chlor-6-cyano-2,2-dimethyl-4-chromanol, 250 mg p-Toluolsulfonsäure und 200 ml Toluol wurde über 7 Stunden am aufgesetzten Wasserabscheider und Rückflußkühler gekocht, das Lösungsmittel abdestilliert und das 7-Chlor-6-cyano-2,2-dimethyl-3,4-chromen in Gegenwart von Wasser zur Kristallisation gebracht. Schmp. 74 - 80 °C.
e) trans-4-Brom-6-cyano-7-chlor-3-hydroxy-2,2-dimethylchroman erhalt man analog der unter 1 c) angegebenen Vorschrift durch Umsetzung von 7-Chlor-6-cyano-2,2-dimethyl-3,4-chromen mit N-Bromsuccinimid und Wasser. Hellgelbe Kristalle aus Diisopropylether, Schmp.: 152 - 155 °C.
f) 6-Cyano-7-chlor-2,2-dimethyl-3,4-epoxychroman erhält man analog der in Beispiel 1 d) angegebenen Vorschrift durch Umsetzung von trans-4-Brom-6-cyano-7-chlor-3-hydroxy-2,2-dimethylchroman mit NaOH in THF. Kristalliner Feststoff, Schmp 129 - 132 °C.
g) 7-Chlor-6-cyano-4-[N-ethylsulfonyl-N-methylamino]-2,2-dimethyl-3-chromanol erhält man analog der in Beispiel 1 e) angegebenen Vorschrift aus 6-Cyano-7-chlor-2,2-dimethyl-3,4-epoxychroman und Ethylsulfonsäure-N-methylamid mit NaH in DMSO. Farblose Kristalle aus Gemisch von Diisopropylether und wenig Ethylacetat. Schmp. 179 - 182 °C.

### Beispiel 3: 3-Acetoxy-7-chlor-6-cyano-4-[N-ethylsulfonyl-N-methylamino]-2,2-dimethylchroman

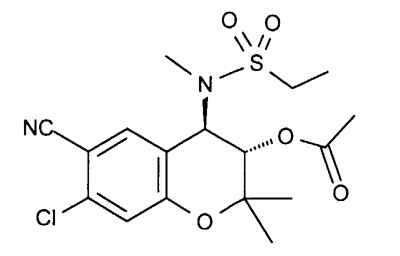

Zu einer Lösung aus 0,6 g (0.0016 M) 6-Cyano-4-[N-ethylsulfonyl-N-methylamino]-7-fluor-2,2-dimethyl-3-chromanol in 8 ml wasserfreiem Pyridin tropft man unter Eiskühlung 1,44 g (0,014 M) Acetanhydrid und läßt die Mischung nach Entfernung des Eisbades auf Raumtemperatur kommen. Man rührt 2 Tage bei Raumtemperatur, destilliert das Lösungsmittel ab und bringt den Rückstand in Gegenwart von Wasser zur Kristallisation. Farbloses kristallines Produkt, Schmp. 157 - 160 °C.

### Beispiel 4: 7-Chlor-4-[N-ethylsulfonyl-N-methylamino]-6-fluor-2,2-dimethyl-3-chromanol

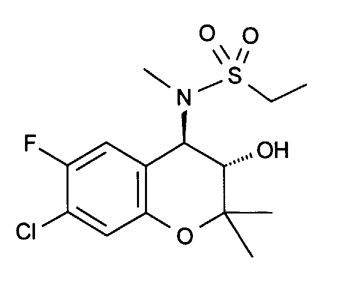

a) 4-Chlor-5-fluor-2-hydroxy-acetophenon
   erhält man analog der in Beispiel 2 a) angegebenen Vorschrift aus 4-Acetoxy-2-chlor-fluorbenzol (dargestellt durch Erhitzen von 3-Chlor-4-fluorphenol in Acetanhydrid, Schmp. 39 - 44 °C) durch Erhitzen mit wasserfreiem Aluminiumchlorid als Friedel-Crafts-Katalysator. Kristalline Substanz, Schmp. 74-75 °C (Gemisch aus n-Heptan und Diisopropylether).
b) 7-Chlor-6-fluor-2,2-dimethyl-4-chromanon
   erhält man analog der in Beispiel 2 b) angegebenen Vorschrift aus 4-Chlor-5-fluor-2-hydroxy-acetophenon und Aceton in Gegenwart von Pyrrolidin als Katalysator. Teilweise kristallisierendes Öl.
c) 7-Chlor-6-fluor-2,2-dimethyl-4-chromanol
   erhält man analog der in Beispiel 2 c) angegebenen Vorschrift durch Reduktion von 7-Chlor-6-fluor-2,2-dimethyl-4-chromanon mit Natriumboranat. Helles, ölig amorphes Produkt.
d) 7-Chlor-6-fluor-2H-2,2-dimethyl-4-chromen
   erhält man analog der in Beispiel 2 d) angegebenen Vorschrift durch säurekatalysierte Wasserabspaltung aus 7-Chlor-6-fluor-2,2-dimethyl-4-chromanol mit p-Toluolsulfonsäure. Braunes öliges Produkt.
e) trans-4-Brom-7-chlor-6-fluor-3-hydroxy-2,2-dimethylchroman
   erhalt man analog der unter 1 c) angegebenen Vorschrift durch Umsetzung von 7-Chlor-6-fluor-2,2-dimethyl-4-chromen mit N-Bromsuccinimid und Wasser. Hellgelbe Kristalle, Schmp.: 97 - 99 °C.
f) 7-Chlor-6-fluor-2,2-dimethyl-3,4-epoxychroman erhält man analog der in Beispiel 1 d) angegebenen Vorschrift durch Umsetzung von trans-4-Brom-7-chlor-6-fluor-3-hydroxy-2,2-dimethylchroman mit NaOH in THF. Amorphes öliges Produkt.
g) 7-Chlor-4-[N-ethylsulfonyl-N-methylamino]-6-fluor-2,2-dimethyl-3-chromanol
   erhält man analog der in Beispiel 1 e) angegebenen Vorschrift aus 7-Chlor-6-fluor-2,2-dimethyl-3,4-epoxychroman und Ethylsulfonsäure-N-methylamid mit NaH in DMSO.
   Hellbeige kristalline Verbindung, Schmp. 188 - 190 °C.

### Beispiel 5: 4-[N-Butyl-N-ethylsulfonylamino]-7-chlor-6-fluor-2,2-dimethyl-3-chromanol

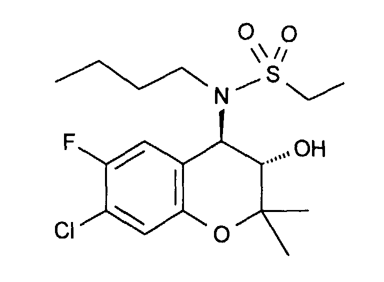

erhält man analog der in Beispiel 1 e) angegebenen Vorschrift aus 7-Chlor-6-fluor-2,2-dimethyl-3,4-epoxychroman und Ethylsulfonsäure-N-butylamid mit NaH in DMSO.

### Beispiel 6: 3-Acetoxy-7-chlor-4-[N-ethylsulfonyl-N-methylamino]-6-fluor-2,2-dimethylchroman

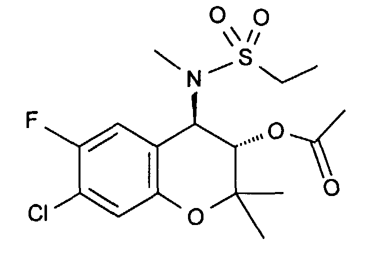

erhält man analog der in Beispiel 3 angegebenen Vorschrift aus 7-Chlor-4-[N-ethylsulfonyl-N-methylamino]-6-fluor-2,2-dimethyl-3-chromanol und Acetanhydrid in Pyridin. Farblose, kristalline Substanz, Schmp. 143 - 144 °C.

### Beispiel 7: 7-Chlor-4-[N-ethylsulfonyl-N-methylamino]-6-fluor-2,2-dimethyl-2H-chromen

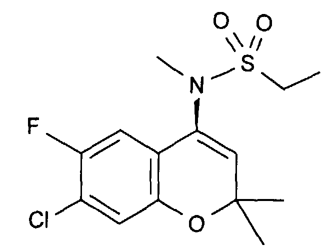

Eine Mischung aus 0,6 g 3-Acetoxy-7-chlor-4-[N-ethylsulfonyl-N-methylamino]-6-fluor-2,2-dimethylchroman, 1,2 ml DBU und 15 ml Toluol wird für 2 Stunden am Rückflußkühler gekocht und das Lösungsmittel abdestilliert. Der Rückstand wird in Ethylacetat gelöst, sodann die organische Phase mit 2 N Salzsäure, mit Wasser und anschließend mit gesättigter Natriumbicarbonat-Lösung gewaschen und das Lösungsmittel abdestilliert. Das viskose Öl wird in Gegenwart von n-Heptan zur Kristallisation gebracht. Schmp. 83 - 84 °C.

### Beispiel 8: 6,8-Dichlor-4-[N-ethylsulfonyl-N-methylamino]-2,2-dimethyl-3-chromanol

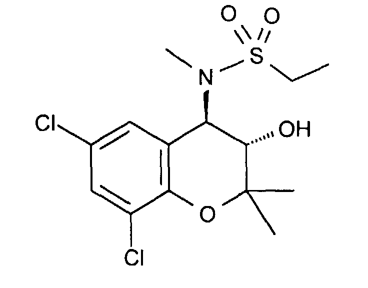

a) 6,8-Dichlor-2,2-dimethyl-4-chromanon
   erhält man analog der in Beispiel 2 b) angegebenen Vorschrift aus 3,6-Dichlor-2-hydroxy-acetophenon und Aceton unter katalytischer Beschleunigung durch Pyrrolidin.
   Dunkel gefärbtes viskoses Öl.
b) 6,8-Dichlor-2,2-dimethyl-4-chromanol
   erhält man analog der in Beispiel 2 c) angegebenen Vorschrift durch Reduktion von 6,8-Dichlor-2,2-dimethyl-4-chromanon mit Natriumboranat. Braunes, ölig amorphes Produkt.
c) 6,8-Dichlor-2H-2,2-dimethyl-3-chromen
   erhält man analog der in Beispiel 2 d) angegebenen Vorschrift durch säurekatalysierte Wasserabspaltung aus 6,8-Dichlor-2,2-dimethyl-4-chromanol mit p-Toluolsulfonsäure.
d) trans-4-Brom-6,8-dichlor-3-hydroxy-2,2-dimethylchroman
   erhalt man analog der unter 1 c) angegebenen Vorschrift durch Umsetzung von 6,8-Dichlor-2H-2,2-dimethyl-3-chromen mit N-Bromsuccinimid und Wasser. Beige gefärbte kristalline Substanz, Schmp.: 46 °C.
e) 6,8-Dichlor-2,2-dimethyl-3,4-epoxychroman
   erhält man analog der in Beispiel 1 d) angegebenen Vorschrift durch Umsetzung von trans-4-Brom-6,8-dichlor-3-hydroxy-2,2-dimethylchroman mit NaOH in THF. Dunkles amorphes öliges Produkt.
f) 6,8-Dichlor-4-[N-ethylsulfonyl-N-methylamino]-2,2-dimethyl-3-chromanol
   erhält man analog der in Beispiel 1 e) angegebenen Vorschrift aus 6,8-Dichlor-2,2-dimethyl-3,4-epoxychroman und Ethylsulfonsäure-N-methylamid mit NaH in DMSO.
   Ölig-amorphes Produkt.

### Beispiel 9: 3-Acetoxy-6,8-dichlor-4-[N-ethylsulfonyl-N-methylamino]-2,2-dimethylchroman

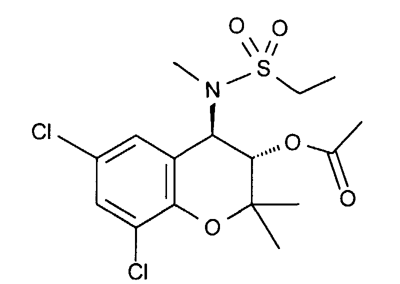

erhält man analog der in Beispiel 3 angegebenen Vorschrift aus 6,8-Dichlor-4-[N-ethylsulfonyl-N-methylamino]-2,2-dimethyl-3-chromanol und Acetanhydrid in Pyridin. Farblose, kristalline Substanz, Schmp. 136-137 °C.

### Beispiel 10: 4-[N-Butyl-N-ethylsulfonylamino]-6,8-dichlor-2,2-dimethyl-3-chromanol

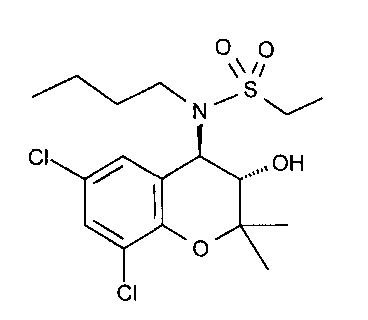

erhält man analog der in Beispiel 1 e) angegebenen Vorschrift aus 6,8-Dichlor-2,2-dimethyl-3,4-epoxychroman und Ethylsulfonsäure-N-butylamid mit NaH in DMSO. Ölig-amorphes Produkt.

## Patentansprüche

1. Chroman-Derivate der Formel I 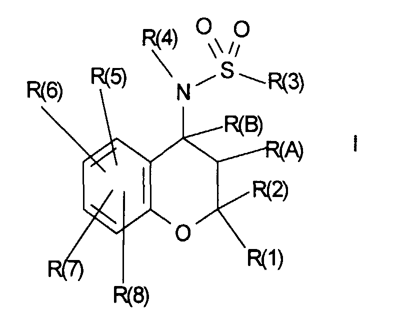 worin bedeuten:
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 2, 3, 4, 5, 6, 7, 8, 9 oder 10 C-Atomen;
R(A) Hydroxy, Alkanoyloxy mit 1, 2, 3, 4, 5 oder 6 C-Atomen oder Alkylsulfonyloxy mit 1, 2, 3, 4, 5 oder 6 C-Atomen;
R(B) Wasserstoff;
oder
R(A) und R(B)
zusammen eine Bindung;
R(3) R(9)-CₙH₂ₙ[NR(11)]ₘ-;
R(9) Wasserstoff oder Cycloalkyl mit 3, 4, 5, 6, 7 oder 8 C-Atomen;
n Null, 1, 2, 3, 4, 5, 6, 7, 8, 9 oder 10;
m Null oder 1;
R(11) Wasserstoff oder Alkyl mit 1, 2, 3, 4, 5 oder 6 C-Atomen;
oder
R(11) und R(9)
gemeinsam eine Alkylengruppe mit 1, 2, 3, 4, 5, 6, 7 oder 8 C-Atomen;
wobei eine CH₂-Gruppe der Gruppe CₙH₂ₙ durch -O-, SO_{Null, 1 oder 2}- oder -NR(10) ersetzt sein kann;
R(10) Wasserstoff, Methyl oder Ethyl;
R(4) R(12)-CᵣH₂ᵣ;
R(12) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
wobei Pyridyl, Thienyl, Imidazolyl oder Phenyl unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
r Null, 1, 2, 3, 4, 5, 6, 7, 8, 9, 10, 11, 12, 13, 14, 15, 16, 17, 18, 19 oder 20;
wobei eine CH₂-Gruppe der Gruppe CᵣH₂ᵣ ersetzt sein kann durch -O-, >CH=CH<, -C≡C-, -CO-, -CO-O-, SO_{Null, 1 oder 2}- oder -NR(10)-;
R(5), R(6), R(7) und R(8)
unabhängig voneinander Wasserstoff, F, Cl, Br, I, Alkyl mit 1, 2, 3, 4, 5 oder 6 C-Atomen, Cycloalkyl mit 3, 4, 5, 6, 7 oder 8 C-Atomen, -CN, -CF₃, -C₂F₅, -C₃F₇, -N₃, -NO₂, -CONR(13)R(14), -COOR(15),
R(16) -CₛH₂ₛ-Y- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(13) und R(14)
unabhängig voneinander Wasserstoff oder Alkyl mit 1, 2 oder 3 C-Atomen;
R(15) Wasserstoff, Methyl, Ethyl, Phenyl oder -CᵤH₂ᵤ-NR(13)R(14); u 2 oder 3;
R(16) Wasserstoff, Cycloalkyl mit 3, 4, 5, 6, 7 oder 8 C-Atomen, -COOR(15), Thienyl, Imidazolyl, Pyridyl, Chinolyl, Isochinolyl, Piperidyl, 1-Pyrrolidinyl, N-Morpholino, N-Methylpiperazino, CF₃, C₂F₅, C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
s Null, 1, 2, 3, 4, 5 oder 6;
Y -S-, -SO-, -SO₂₋, -CO-, -SO₂-NR(10)-, -O-, -NR(10)- oder -CO-NR(10);
jedoch mit der Bedingung, daß zwei der Substituenten R(5), R(6), R(7) und R(8) nicht Wasserstoff sind;
und deren physiologisch verträgliche Salze.

2. Verbindungen der Formel I nach Anspruch 1, in der bedeuten:
R(1) und R(2)
unabhängig voneinander Wasserstoff, Alkyl mit 1, 2 oder 3 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 4 oder 5 C-Atomen;
R(A) Hydroxy oder Acetoxy;
R(B) Wasserstoff;
oder
R(A) und R(B)
zusammen eine Bindung;
R(3) R(9)-CₙH₂ₙ[NR(11)]ₘ-;
R(9) Wasserstoff;
n Null, 1, 2, 3, 4, 5 oder 6;
m Null oder 1;
R(11) Wasserstoff oder Alkyl mit 1, 2, 3 oder 4 C-Atomen;
R(10) Wasserstoff, Methyl oder Ethyl;
R(4) R(12)-CᵣH₂ᵣ;
R(12) Wasserstoff, Cycloalkyl mit 3, 4, 5, 6, 7 oder 8 C-Atomen, CF₃, Pyridyl oder Phenyl,
wobei Pyridyl und Phenyl unsubstituiert sind oder substituiert mit 1 oder 2 Substituenten ausgewählt aus der Gruppe bestehend aus F, Cl, CF₃, Sulfamoyl und Methylsulfonyl;
r Null, 1, 2, 3, 4, 5, 6, 7, 8, 9 oder 10;
wobei eine CH₂-Gruppe der Gruppe CᵣH₂ᵣ ersetzt sein kann durch -O-, -CO-, -CO-O-, SO_{Null, 1 oder 2}-;
R(5), R(6), R(7) und R(8)
unabhängig voneinander Wasserstoff, F, Cl, Br, Alkyl mit 1, 2, 3 oder 4 C-Atomen, -CN, -CF₃, -NO₂, -CONR(13)R(14), -COOR(15), R(16)-CₛH₂ₛ-Y- oder Phenyl,
das unsubstituiert ist oder substituiert mit 1 oder 2 Substituenten ausgewählt aus der Gruppe bestehend aus F, Cl, und CF₃;
R(13) und R(14)
unabhängig voneinander Wasserstoff oder Alkyl mit 1, 2 oder 3 C-Atomen;
R(15) Methyl, Ethyl, Phenyl oder -CᵤH₂ᵤ-NR(13)R(14);
u 2 oder 3;
R(16) Wasserstoff, Cycloalkyl mit 5 oder 6 C-Atomen, CF₃, C₂F₅, C₃F₇ oder Phenyl,
das unsubstituiert ist oder substituiert mit 1 oder 2 Substituenten ausgewählt aus der Gruppe bestehend aus F, Cl, Br, CF₃, Methyl, Methoxy, Sulfamoyl und Methylsulfonyl;
s Null, 1, 2, 3 oder 4;
Y -S-, -SO-, -SO₂₋, -CO-, -SO₂-NR(10)-, -O-, -NR(10)- oder -CO-NR(10);
jedoch mit der Bedingung, daß zwei der Substituenten R(5), R(6), R(7) und R(8) nicht Wasserstoff sind.

3. Verbindungen der Formel I nach Ansprüchen 1 oder 2, in welcher bedeuten:
R(1) und R(2)
unabhängig voneinander Alkyl mit 1, 2 oder 3 C-Atomen;
oder
R(1) und R(2)
gemeinsam eine Alkylenkette mit 4 oder 5 C-Atomen;
R(A) Hydroxy;
R(B) Wasserstoff;
oder
R(A) und R(B)
zusammen eine Bindung;
R(3) Alkyl mit 1, 2 oder 3 C-Atomen, Dimethylamino oder Diethylamino;
R(4) R(12)-CᵣH₂ᵣ; R(12) Wasserstoff, Cycloalkyl mit 5 oder 6 C-Atomen oder CF₃;
r Null, 1, 2, 3, 4, 5, 6, 7 oder 8;
wobei eine CH₂-Gruppe der Gruppe CᵣH₂ᵣ ersetzt sein kann durch -O-, -CO-, -CO-O-, SO_{Null, 1 oder 2}-;
R(5), R(6), R(7) und R(8)
unabhängig voneinander Wasserstoff, F, Cl, Br, Alkyl mit 1 oder 2 C-Atomen, -CN, -NO₂, -COOR(15), R(16)-CₛH₂ₛ-Y- oder Phenyl,
das unsubstituiert ist oder substituiert mit 1 oder 2 Substituenten ausgewählt aus der Gruppe bestehend aus F und Cl;
R(15) Methyl, Ethyl, Phenyl oder -CᵤH₂ᵤ-NR(13)R(14);
u 2 oder 3;
R(13) und R(14)
unabhängig voneinander Wasserstoff oder Alkyl mit 1, 2 oder 3 C-Atomen;
R(16) Wasserstoff, CF₃ oder Phenyl,
s Null, 1, 2, 3 oder 4;
Y -S-, -SO-, -SO₂₋, -CO-, -SO₂-NR(10)-, -O-, -NR(10)- oder -CO-NR(10);
R(10) Wasserstoff oder Methyl;
jedoch mit der Bedingung, daß zwei der Substituenten R(5), R(6), R(7) und R(8) nicht Wasserstoff sind.

4. Verwendung einer Verbindung der Formel I nach Anspruch 1 zum Herstellen eines Medikaments zum Blockieren des K⁺ Kanal, der durch cyclisches Adenosinmonophosphat (cAMP) geöffnet wird.

5. Verwendung einer Verbindung der Formel I nach Anspruch 1 zum Herstellen eines Medikaments zum Inhibieren Magensäuresekretion.

6. Verwendung einer Verbindung der Formel I nach Anspruch 1 zum Herstellen eines Medikaments zur Behandlung von Ulcera des Magens und des intestinalen Bereiches, insbesondere des Duodenums.

7. Verwendung einer Verbindung der Formel I nach Anspruch 1 zum Herstellen eines Medikaments zur Behandlung der Refluxoesophagitis.

8. Verwendung einer Verbindung der Formel I nach Anspruch 1 zum Herstellen eines Medikaments zur Behandlung von Durchfall-Erkrankungen.

9. Verwendung einer Verbindung der Formel I nach Anspruch 1 zum Herstellen eines Medikaments zur Behandlung und Verhinderung aller Typen von Arrhythmien einschließlich ventrikulärer und supraventrikulärer Arrhythmien.

10. Verwendung einer Verbindung der Formel I nach Anspruch 1 zum Herstellen eines Medikaments zur Behandlung und Verhinderung aller Typen von Arrhythmien einschließlich atrialer Arrhythmien.

11. Verwendung einer Verbindung der Formel I nach Anspruch 1 zum Herstellen eines Medikaments zur Kontrolle von Reentry-Arrhythmien und zur Verhinderung des plötzlichen Herztodes infolge von Kammerflimmerns.

12. Heilmittel, enthaltend eine wirksame Menge einer Verbindung der Formel I nach einem der Ansprüche 1 bis 4.
